# EUROPEAN PATENT SPECIFICATION

(11) **EP 1 896 019 B1**
(45) Date of publication and mention of the grant of the patent: **02.09.2009**
(21) Application number: 06772240.5
(22) Date of filing: 06.06.2006
(51) Int. Cl.: A61K 31/4365

(54) **FORMULATION OF A THIENOPYRIDINE PLATELET AGGREGATION INHIBITOR**
FORMULIERUNG EINES THIENOPYRIDIN-THROMBOZYTENAGGREGATIONSHEMMERS
FORMULATION D'UNE THIENOPYRIDINE, COMME INHIBITEUR DE L'AGREGATION PLAQUETTAIRE

(30) Priority: 10.06.2005 US 689183 P
(43) Date of publication of application: 12.03.2008
(73) Proprietor: ELI LILLY AND COMPANY, Indianapolis IN 46285 (US)
(72) Inventor: DZIENNIK, David, Brian, Fortville, Indiana 46040 (US); EDELMAN, Tamara, Beth, Noblesville, Indiana 46060 (US); OREN, Peter, Lloyd, Fishers, Indiana 46038 (US); TERNIK, Robert, Louis, Fishers, Indiana 46038 (US)
(74) Representative: Commander, Paul Martin Brial
(86) International application number: PCT/US2006/021860
(87) International publication number: WO 2006/135605

(56) References cited:
- US-A1- 2003 134 872
- ANONYMOUS: "Die Folie macht den Unterschied" INTERNET ARTICLE, [Online] February 2004 (2004-02), XP002406026 Retrieved from the Internet: URL:http://www.uhlmann.de/fileadmin/redakt eur/pdf/d/PVC-Hartfolie_neu_d.pdf>
- ALLINSON JENNIFER G ET AL: "The effects of packaging on the stability of a moisture sensitive compound" INTERNATIONAL JOURNAL OF PHARMACEUTICS (KIDLINGTON), vol. 221, no. 1-2, 19 June 2001 (2001-06-19), pages 49-56, XP002406675 ISSN: 0378-5173
- FERRIER D ET AL: "The control of packaging materials part II - Plastic and other materials" S.T.P. PHARMA PRATIQUES 2004 FRANCE, vol. 14, no. 2, 2004, pages 221-256, XP001248015 ISSN: 1157-1497

## Description

### Field of the Invention

The invention relates to a novel formulation of Prasugrel.

### Background of the Invention

Thienopyridines such as Ticlopidine and Clopidogrel (sold as Plavix ®. registered trademark of Sanofi-Aventis S.A) have been used for the treatment of thrombosis and related diseases. Clopidogrel in particular has found widespread use compared to the older ticlopidine.

Prasugrel is a next generation thienopyridine currently undergoing clinical development for the treatment of thrombosis and/or related diseases including as an adjunct to percutaneous coronary intervention procedures.

US Patent 5,288,726 discloses and claims tetrahydrothienopyridine derivatives including 2-Acetoxy-5-(α-cycloprpylcarbonyl-2-fluorobenzyl)-4,5,6,7-tetrahydrothieno[3,2-c]pyridine.

US patent 6, 693,115 B2 discloses and claims the hydrochloric acid and maleic acid salts of 2-Acetoxy-5-(α-cycloprpylcarbonyl-2-fluorobenzyl)-4,5,6,7-tetrahydrothieno[3,2-c]pyridine. The HCl and maleate salt forms provide unexpected and unobvious improvements in their efficacy and stability profiles compared to other salts and also compared to the free base molecule. The HCl salt of 2-Acetoxy-5-(α-cycloprpylcarbonyl-2-fluorobenzyl)-4,5,6,7-tetrahydrothieno[3,2-c]pyridine is also known as Prasugrel. Prolonged exposure of the HCl salt (prasugrel) to air and moisture results in some degradation.

Therefore, there is a need for further improvements in the stability, shelf life and therefore long term efficacy of individual doses of prasugrel.

### Summary of the Invention

The present invention provides a formulation comprising a therapeutically effective amount of the compound of formula I 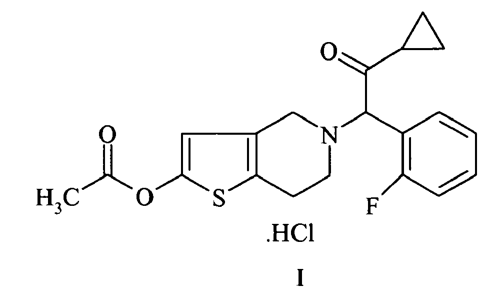 packaged in an air and moisture impervious gas-inerted blister pack.

The present invention further provides a formulation of compound I comprising a tablet, caplet, capsule or other solid formulation of the compound of formula I packaged in an air and moisture impervious gas-inerted blister pack.

The present invention provides an improved formulation of the compound of formula I comprising a therapeutically effective amount of a tablet, caplet, capsule or other solid formulation of the compound of formula I packaged in an air and moisture impervious gas-inerted blister pack.

The present invention relates to a method of improving the stability and shelf life of the compound of formula I comprising packaging tablet(s), caplet(s) or capsule(s) or other solid formulation of the compound of formula I in gas inerted aluminum foil blister pack(s).

The present invention relates to a formulation of the compound of formula I comprising a tablet, caplet, capsule or other solid formulation of the compound of formula I which has been packaged in an air and moisture-impervious gas-inerted blister pack for use as a medicament.

The present invention relates to a formulation of the compound of formula I comprising a tablet, caplet, capsule or other solid formulation of the compound of formula I which has been packaged in an air and moisture impervious gas-inerted blister pack for use in the treatment and/or prevention of thrombosis, acute coronary syndrome, ACS-MM, stroke, cerebrovascular aneurysyms, and high risk vascular diseases.

The present invention relates to a pharmaceutical formulation of a compound of formula I which has been packaged in a nitrogen-inerted aluminum foil blister pack in combination with other cardio protective agents for use in the treatment of Cardiovascular Diseases.

The present invention relates to a method for improving the stability and shelf life of a pharmaceutical composition comprising a compound of formula I wherein individual tablet(s), caplet(s) or capsule(s) of the compound of formula I is/are packaged in nitrogen-inerted aluminum foil blister packs.

The present invention provides a formulation of a compound for formula I comprising a therapeutically effective amount of the compound of formula 1 from about 5 mg to about 60 mg base equivalent packaged in a gas-inerted aluminum foil blister pack.

The present invention provides a process for the manufacture of a compound of formula I comprising the steps of:
a. preparing tablets, caplets or capsules of the compound of formula I, and
b. packaging said tablets, caplets or capsules of the compound of formula I in gas inerted aluminum foil blister packs.

The present invention provides an article of manufacture comprising a compound of formula I packaged in an air and moisture impervious gas-inerted blister pack.

### Detailed Description

As used herein the term "Prasugrel" means the compound of formula I as shown. While the compound is also named CS-747HCl, and Prasugrel HCl here and elsewhere, these terms mean one and the same thing, the compound of formula I as shown.

The term, "Cardiovascular Diseases" refers to diseases treatable, preventable, or able to be ameliorated by treatment with a compound of formula I and/or by performance of cardiac interventional procedures including coronary (PCI) and non-coronary interventions. Examples of cardiovascular diseases encompassed by the invention include coronary occlusion, restenosis, stroke, acute coronary syndrome (ACS), ACS with medical management (ACS-MM), high risk vascular diseases (HRVD), cerebro vascular aneurysm (CVA), congestive heart failure, cardiac alternation, ventricular aneurysm, neural aneurysm, myocardial infarction, cardiac arrest, cardiac dysrhythmia including atrial fibrillation, cardiac edema, cardiac dyspnea, cardiac failure, tachycardia, cardiac hemoptysis, cardiac incompetence, cardiac murmur, cardiac syncope, cardiac tamponade, cerebrovascular disease and/or peripheral artery disease.

"Administering" as used herein refers to an oral administration of the compound of formula I including buccal, sublingual and other forms of oral administration, which allow for the compound of formula I to perform its intended function of treating and/or preventing the occurrence or recurrence of Cardiovascular Diseases independently or as part of a combination therapy (treatment) with an interventional procedure such as a PCI procedure or as part of a combination treatment with other cardio-protective agents. Such administration by virtue of the combination treatment includes the performance of a PCI procedure e.g. the implantation of stent, or performance of balloon angioplasty.

The term "treatment" as used herein refers to the amelioration, inhibition, prevention of occurrence or recurrence, reduction in severity or effect of cardiovascular diseases including but not limited to restenosis, acute coronary syndromes (ACS) including medically managed ACS, myocardial infarction, cercbro vascular aneurysm, and high risk vascular diseases by the use of a compound of formula I singly or in combination with other cardio-protective agents or as an adjunct to an interventional procedure such as PCI or other interventional procedure.

The term "therapeutically effective amount" as used herein refers to the amount of a compound of formula I necessary or sufficient in single or multiple units to treat the particular Cardiovascular Disease in a treatment regimen comprised of a compound of formula I as prescribed by a qualified treating physician or as approved by applicable regulatory authorities.

The therapeutically effective amount may vary depending on factors known to one of skill in the art (a qualified prescriber) including for example, the optional combination of compound I with aspirin or other cardio-protective agent or interventional procedure such as PCI, the use of drug coated stents, mode and regimen of administration, the size of the subject, genetic or behavioral predisposition to Cardiovascular Diseases or the severity and recurrence thereof. One of skill in the art would be able to consider these and related factors to make the appropriate determination regarding the therapeutically effective amount for a particular patient.

The phrase "other cardio protective agents" as used herein refers to therapeutic agents that have been proven and approved to provide beneficial effects (treatment and/or prevention of occurrence or recurrence) to a patient afflicted with or susceptible to Cardiovascular Diseases. Example of cardio-protective agents include but are not limited to aspirin, effective GPIIb/IIIa inhibitors, effective statins such as HMG-CoA reductase inhibitors, super statins, acyl CoA-cholesterol O-acyltransferase (ACAT) inhibitors, effective anticoagulants, effective thienopyridines, and other effective lipid modifying agents.

The phrase "pharmaceutically acceptable carrier" refers to any substance or medium co-formulated with the compound of formula 1 and which allows the compound to perform its intended function. Examples of such carriers include solutions, solvents, dispersion media, delay agents, emulsions, microparticles and the like for combination therapies.

The phrases "combination therapy," "combination treatment," "in conjunction with," "combination of a compound of formula I and stent," and "in conjunction with a PCI procedure" if and as used herein are synonymous and indicate that a patient who is a candidate for a PCI procedure or other interventional procedure is administered a therapeutically effective dose(s) of a compound of formula I or a pharmaceutically acceptable salt, prodrug, active metabolitc, racemate or enantiomer thereof, optionally in combination with aspirin at a reasonable period of time prior to and/or after PCI or other interventional procedure. A reasonable period of time for administering the compound of formula I, optionally with aspirin, prior to PCI or other interventional procedure may be up to about sixty days prior and may include no prior administration. The purpose of the prior administration is to achieve an on-going beneficial effect plus a rapid onset of an effect on platelet function prior to the intervention procedure, and over and above the rapid onset characteristic of a compound of formula I, without prior treatment (loading dose), thereby maximizing the potential benefit to the patient. The dosing of a compound of formula I prior to an interventional procedure such as stenting or balloon angioplasty may not be practical or necessary in emergency situations. For the purpose of this invention a reasonable period after PCI or other interventional procedure, for conjunctive treatment with a compound of formula I, may be a period of from about 5 days to about 700 days and preferably from about 30 days to about 365 days. Ultimately, the precise period of therapy according to this invention is a determination to be made by the treating or attending physician and tailored to the particular patient.

The phrase "air and moisture impervious" as used herein means materials of appropriate thickness known to one of skill in the art or ascertainable with minimal experimentation that when sealed within specifications are likely to substantially and significantly prevent air and moisture entry and egress. One of skill in the art is aware that absolute imperviousness may be difficult to achieve and that the inventors believe that the phrase "air and moisture impervious" material or blister pack is used comparatively based on the knowledge of one skilled in the art that some materials are less impervious to air and moisture than others and that absolute imperviousness is difficult to attain. A material that is both air and moisture impervious is preferred. Examples of air and moisture impervious materials include aluminum, PCTFE (Aclar^{®}) and Aclar^{®}-EVOH. Aluminum foil blister packs are most preferred.

The phrase "gas-inerted" as used herein means that a gas that is inert to the tablet, capsule, caplet or other solid formulation surrounds the available cavity or space other than that occupied by the tablet, caplet, or capsule in a blister pack. The gas may be an inert gas or other gas that does not adversely affect (react with) the tablet, caplet or capsule. Examples of gases useful as inerting gases include CO₂, argon, nitrogen, neon, krypton, and CO (in non-lethal pharmaceutically acceptable quantities). More preferred as a gas useful for the practice of the invention is nitrogen or argon. Most preferred is nitrogen.

The phrases "predominantly," and "predominance of an inert gas" as used herein are synonymous and are intended to mean that the volume of space surrounding the tablet, caplet or capsule in the blister pack cavity is essentially or nearly or as much as practically possible completely filled with nitrogen or other inert gas. The effect of said "predominance of an inert gas" is that oxygen content is reduced to about less than 2% to 4%.

The term "base equivalent" as used herein conveys its ordinary meaning, i.e. amount of the compound of formula I (the HCl salt) that is equivalent to the base form. One of skill in the art is able to make the conversion, and sample equivalent amounts are shown in the examples.

The term "other solid formulation" as used herein include fast disintegrating, fast dissolving, quick release or other approved or approvable solid presention of a drug known to one of skill in the art.

The term "formulation" as used herein includes its ordinary meaning and also includes the pharmaceutically prepared compound of formula I and the packaging of said compound of formula I according to the present invention. Thus a compound of formula I formulated either as the tablet, caplet, capsule, slow release or fast disintegrating (dissolving) form or other solid form packaged in a gas-inerted aluminum foil blister pack is a formulation for the purpose of the present invention. Likewise, a compound of formula I formulated either as the tablet, caplet, capsule, slow release or fast disintegrating (dissolving) form or other solid form packaged in a gas-inerted aluminum foil blister pack is an article of manufacture for the purpose of the present invention.

### Preferred Embodiments of the Invention

One embodiment of the present invention is the provision of a pharmaceutical formulation comprising a compound of formula I wherein individual tablets, caplets or capsules of said compound are packaged in an air and moisture impervious material containing an inert gas for the purpose of improving the stability and/or extending shelf life.

In a preferred embodiment the present invention provides a formulation of the compound of formula I wherein tablets, caplets or capsules containing the compound of formula I are packaged in aluminum foil blister packs in an atmosphere comprised predominantly of an inert gas.

In another preferred embodiment the tablets, caplets or capsules of the compound of formula I are packaged in a blister pack(s) containing a gas selected from the group consisting of nitrogen, helium, neon, argon, carbon dioxide, and carbon monoxide.

In a more preferred embodiment the tablets, caplets or capsules of the compound of formula I are packaged in blister pack(s) inerted with a gas selected from the group consisting of nitrogen, helium, and argon. In a most preferred embodiment, the tablets, caplets or capsules of the compound of formula I are packaged in nitrogen-inerted blister pack(s). Thus a most preferred formulation comprises tablets, caplets or capsules of the compound of formula I packaged in nitrogen-inerted aluminum foil blister pack(s).

In another preferred embodiment, the compound of formula I packaged in an air and moisture impervious nitrogen inerted aluminum blister pack is adapted for use in treating ACS, ACS with medical management (ACS-MM) , Stroke, and HRVD singly or in combination with other cardio-protective agents or as an adjunct to an interventional procedure such as PCI or other interventional procedure.

The compound of formula I, analogs, salts, solvates, and enantiomer thereof may be prepared by a variety of methods, including methods described in portions or all of the disclosures of U.S. Patent Nos. 5,288,726 and 6,693,115B2. In particular, US patent 6,693,115 B2 discloses and claims the hydrochloric acid salt of 2-acetoxy-5-(α-cyclopropylcarbonyl-2-fluorobenzyl)-4,5,6,7-tetrahydrothieno[3,2-c]pyridine), the compound of formula I, also known as Prasugrel.

A solid oral dosage form can be prepared using a variety of pharmaceutically acceptable excipients, well known to one skilled in the art. Normally, one or more excipients would be selected from each of the following categories;
(a) Diluents such as but not limited to mannitol, lactose monohydrate, pregelatinized starch or microcrystalline cellulose.
(b) Disintegrants such as but not limited to croscarmellose sodium, low substituted hydroxypropyl cellulose or sodium starch glycolate.
(c) Binders including but not limited to hydroxypropyl methylcellulose and hydroxypropyl cellulose. For tablet applications, a lubricant would also be recommended such as but not limited to magnesium stearate, stearic acid, and glyceryl behenate.

If a tablet is produced, it is often desirable to film coat the resulting tablet to provide a pharmaceutically acceptable appearance and to make said tablet easier to swallow. Commercial suppliers such as, for example, Colorcon Inc.(USA), produce a variety of film coating systems containing polymers, plasticizers and pigments that can be mixed with water and sprayed onto the tablets in a side vented coating pan. A particularly preferred system is marketed as Opadry II^{®} and this film coating system (containing the additive lactose monohydrate) is especially useful in film coating debossed tablets.

Conceivably, prasugrel could be blended with one or more excipients described above and filled into capsules or compressed into tablets. In order to improve the flow properties, it might be desirable to pass these blends through a roller compactor or other equipment to produce a more flowable material.

Because of the stability properties of prasugrel (susceptibility to hydrolysis and oxidation), certain excipients- most notably povidone and crospovidone (usually containing trace peroxides) and manufacturing processes (e.g. wet granulation) would not be recommended. Gas inerted aluminum foil blister packaging is an advantageous and attractive solution to the problem. To further enhance product stability once manufactured, unit dose blister packaging, in particular, gas inerted aluminum foil blister packaging is an advantageous solution to the problem.

Unit dose blister packaging is a convenient presentation for patients and health care providers and these cavities and packages can be prepared from a number of film forming materials. Most commonly, blisters are prepared from films comprised of PVC, PCTFE, and other additives designed to allow the cavity to be formed by heating and tooling designed to create a cavity to hold the finished solid oral dosage form (capsule, gelcap or tablet). After the dosage form is filled into the formed cavities, a foil backing composite is applied to the top of the cavities and the backing is sealed to the blister film by the application of appropriate amount of heat. One of skill in the art is able to effect the above procedure including determining the appropriate amount of heat with minimal experimentation.

Another type of unit dose blister packaging consists of cold form aluminum blisters. In these cases, a heavier gauge of foil with appropriate additives is formed without the application of heat into a cavity to hold the solid oral dosage form. After the dosage form is filled into the formed cavities, a foil backing composite is applied to the top of the cavities and the backing is sealed to the cavities by the application of appropriate heat. One significant advantage of this type of blister is that the aluminum foil is virtually impervious to moisture and oxygen and thus may be particularly suited to packaging dosage forms of Prasugrel (compound of formula I) requiring greater protection from moisture and air. An air and moisture impervious gas-inerted blister pack having aluminum cavity and aluminum foil covering is most preferred.

The introduction of an inert atmosphere into the blister cavity can be accomplished by various means. In one instance, a vacuum may be used to evacuate the air from the formed cavities and the tablets then filled into the cavities in a chamber constructed over the blistering machine such that the atmosphere can be controlled by introduction of an inert gas thereby effectively minimizing the oxygen content.

Alternatively, a gas purging station could be placed on the blistering machine to introduce pressurized inert gases into the cavities containing tablets just prior to the sealing station. By controlling the pressure of the gas purging station, the atmosphere containing oxygen can be forced out of the cavity, effectively reducing the oxygen content of the cavity containing the dosage form by predominantly filling the cavity with inert gas.

Alternatively, gas-inerting may be accomplished by injecting a controlled amount of a liquified inert gas into the blister cavity just prior to the sealing station. As the gas heats up and expands, oxygen may be effectively reduced by displacement.

### Demonstration of the Invention

The stability of tablets, capsules or caplets of the compound of formula I is affected by factors including age (length of storage), and storage conditions, such as for example, temperature and relative humidity. The proper storage conditions ensure an extended shelf life during which the potency of the tablets, caplets or capsules is more likely to be within recommended and/or approved specification limits thereby ensuring the chemical and pharmacodynamic integrity of the tablets, caplets or capsules administered to patients. Studies have shown that despite the improvements associated with the HCl salt of CS-747 vis-à-vis stability, etc (see U.S. Patent 6,693,115B2) there remains room for improvement. Specifically, it is now known that stored tablets containing the compound of formula I degrade by both hydrolytic and oxidative pathways. It is also believed that there are crossovers between these degradation pathways wherein intermediates or products of certain steps in one pathway may inter-convert or be kinetically accelerated or hindered by the concentration of product (or intermediate), air or moisture from the environment or the other pathway. A proposed schematic of degradation pathways as currently postulated is shown below in Schemes 1 and 2.

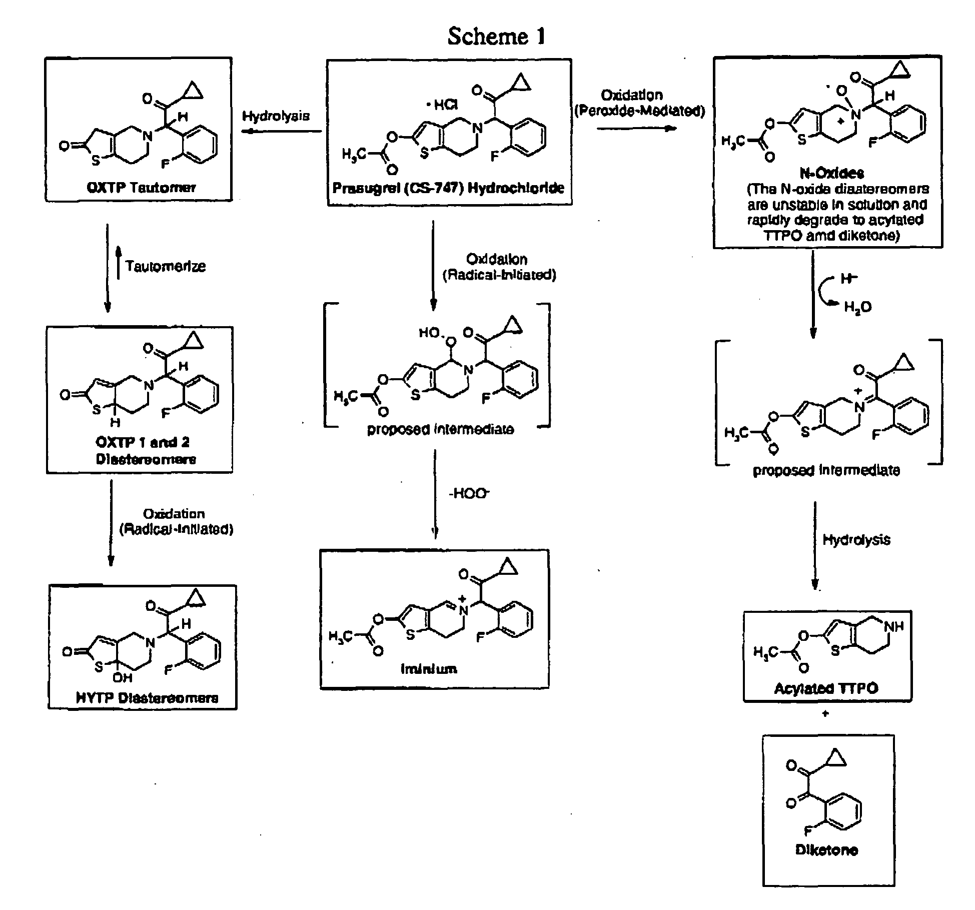

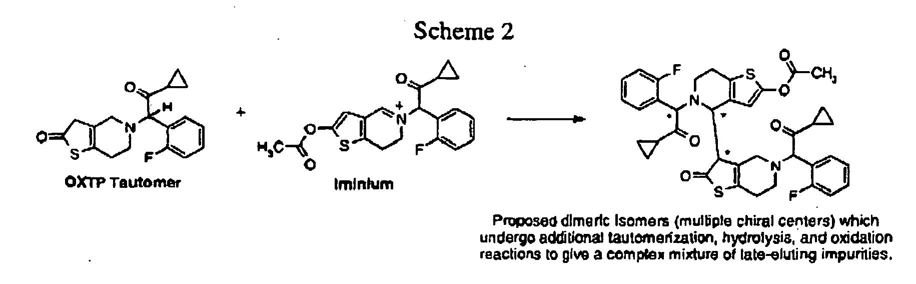

The inventors have been able to individually track hydrolytic degradation products OXTP1 and OXTP2, along with the oxidative degradation products Diketone and HYTP. As shown in Scheme 2, two primary degradation products, OXTP tautomer and iminium, are believed to react with each other to give a mixture of dimeric isomers. These dimeric isomers then can react further resulting in a complex mixture of products that have been termed "late eluting impurities" or LEIs. The level of any individual LEI is insignificant; however, when measured collectively, the amount of degradation represented by the LEIs is significant. The inventors have discovered an improved formulation by tracking the amounts of these degradation products over time under controlled temperature and humidity conditions using different packaging methods. The ideal formulation would result in minimizing the amount of all degradation products over a longer time period. However, because of the interplay of pathways of degradation and inter-conversion between degradation products and pathways, the next best possibility is to discover a packaging or formulation that affords the least change in potency. In other words, a preferred objective is to discover a formulation that affords a composite reduction over time in most of the degradation products thereby satisfying a hitherto unmet need. Also preferred is a formulation of prasugrel comprising a packaging method/process that harnesses the potential advantage of "controlling" the mix of degradation products to produce a more favorable distribution. For example, it may be desirable to have less LEI's and more OXTP 1 and 2. OXTP 1 and 2 are (1) better "known" entities, (2) have been qualified by toxicological studies, (3) have specification limits, and (4) can be quantitated in the related substances method. The inventors have achieved the first objective of a general reduction in impurity profiles allowing for improved stability and longer shelf life. Invention have also achieved the additional objective of "controlling" the mix of degradation products to favor reduction in the amounts of LEI's which are less well-defined or unknown, largely uncharacterized, and for which specified limits have not been set.

The inventors compared the effect of packaging materials and methods on the stability of a drug product containing the compound of formula I. The materials and methods compared include (1) nitrogen inerted blisterpacks comprised of 2.0 mil PCTFE (polychlorotrifluoroethylene) containing blister material and aluminum foil lidding; (2) nitrogen inerted blister packs comprised of 2.0 mil PCTFE/ethyl vinyl alcohol (EVOH) combination blister material and aluminum foil lidding; (3) non-inerted blister packs comprised of cold form aluminum foil blister material with aluminum foil lidding; (4) nitrogen-inerted blister packs comprised of cold form aluminum foil blister material with aluminum foil lidding; and (5) a 50 count, 75-mL bottle with a combination silica gel and carbon desiccant pack. A general packaging description and results for each described package configuration are described below.

A tablet formulation containing 12.5 mg of the compound of formula I was provided for packaging into 4 separate unit dose blister pack configurations, as described above. The blister-packed tablets were then placed into controlled environment chambers having the following conditions: 25 °C at 60% relative humidity, 30 °C at 65% relative humidity, and 40 °C at 75% relative humidity. Samples were removed from these controlled chambers at various elapsed times from their initial placement in the controlled environments. The samples were submitted for chemical analysis to assess changes in potency, total related substances (TRS), OXTP1, OXTP2, Diketone, HYTP and Late Eluting Impurities (LEI). Data from the various blister packed materials along with data for the same lot of tablets packaged in an HDPE bottle with desiccant are presented below.

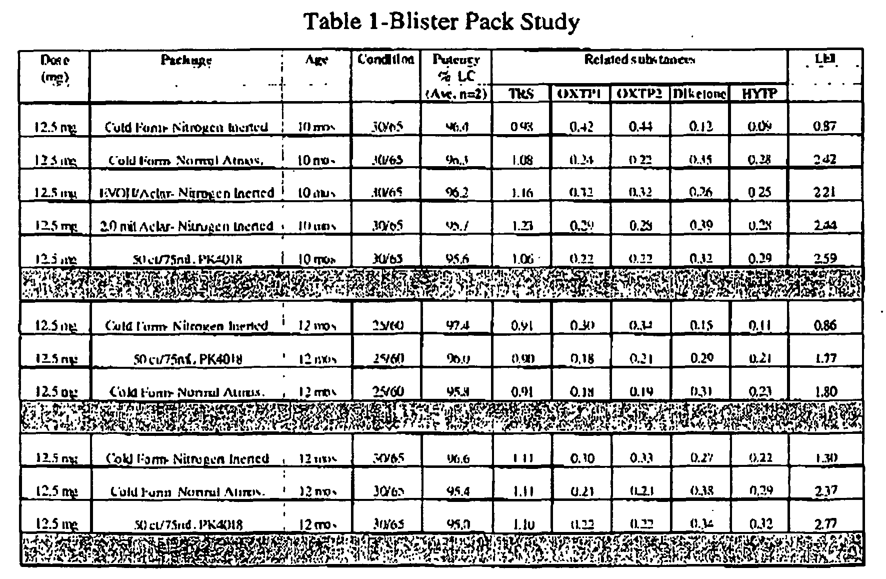

Table 1 shows that the potency of the tablets stored in nitrogen inerted cold form aluminum foil blister packs was higher than that of tablets stored in the other packages that were incapable of preserving a low oxygen environment or which were packaged under normal atmospheric conditions. This was true for each of the storage conditions (25 °C, 60%RH and 30 °C, 65%RH). This trend was true at 10 and 12 months.

Late Eluting Impurities is a combination of peaks the identities of which have not been determined. Advantageously and unexpectedly, the present invention (particularly, the use of cold form nitrogen inerted aluminum foil blister packs) results in a significant reduction of the percentage of these combined late eluting impurities (LEI's) or degradation products. The LEI's in tablets stored in nitrogen inerted cold form aluminum foil blisters were substantially lower than for tablets stored in the other packages that were incapable of maintaining a low oxygen environment or which were packaged under normal atmospheric conditions. This was true for each of the storage conditions (25 °C, 60%RH, 30 °C, 65%RH and 40 °C, 75%RH (not shown)). During the execution of this study, empty blisters were produced with nitrogen inerting using the three blister materials; 2.0 mil PCTFE, EVOH/PCTFE and cold form aluminum blisters. At representative intervals, the oxygen content of these empty blisters was measured to determine the impact of storage time and conditions on this parameter. Representative results are presented below in figure 1:

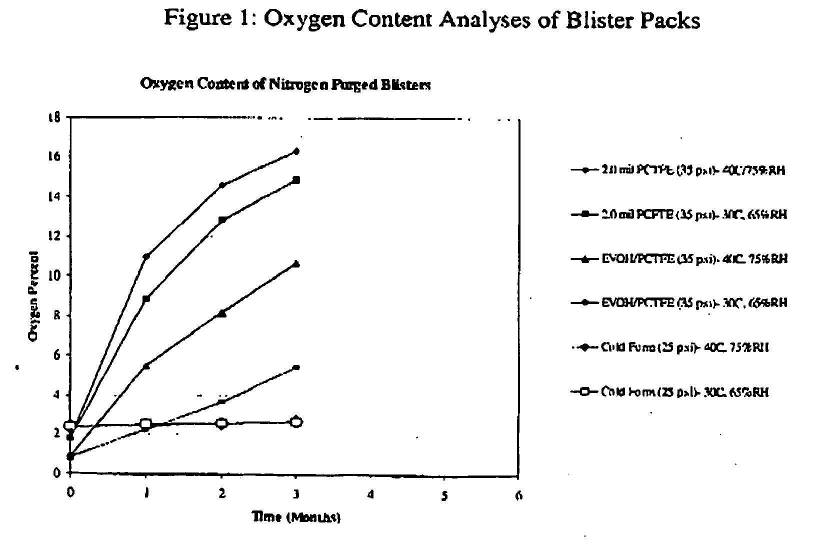

Based on the inability of the two clear blister films (2.0 mil PCTFE and EVOH/PCTFE) to maintain a low oxygen environment, these blisters (2.0 mil PCTFE and EVOR/PCTFE) were not assayed at some of the later timepoints in this study. This study showed that the cold form nitrogen inerted aluminum foil blister packs at 25 psi, 30 °C, and 65% RH contained the least amount of oxygen over time.

In a subsequent formulation stability study, a tablet formulation containing 12.5 mg of the compound of formula I was provided for packaging into foil pouches that were inerted with gases containing known concentrations of oxygen in nitrogen to further elucidate the impact of oxygen concentration on the formation of impurities in the formulation. The results of this study referred to herein as Multi-Vac study are presented in the following Table 2:

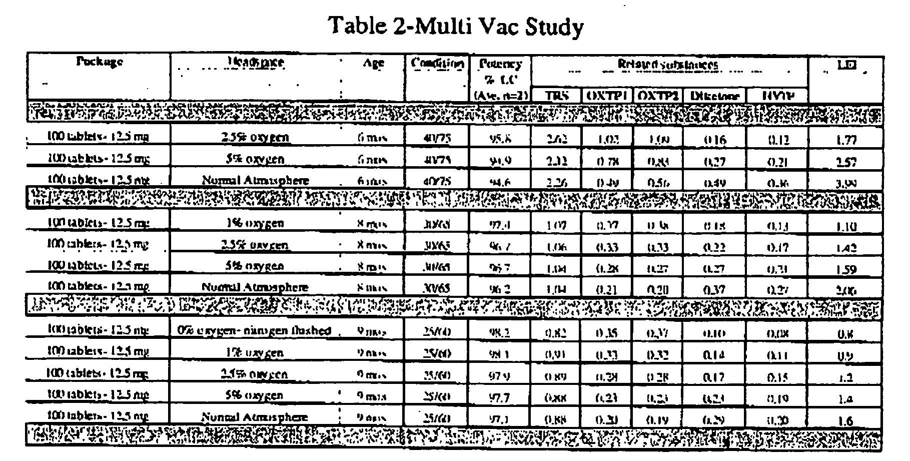

As shown in Table 2, the potency of tablets stored in low oxygen environments such as those achieved with the cold form nitrogen-inerted aluminum foil blister packs was consistently higher than the potency for tablets packaged under normal atmospheric conditions or at higher oxygen environments (as shown in table above). This was true for each of the stability (tested) conditions (25 °C, 60%RH, 30 °C, 65%RH and 40 °C, 75%RH).

The LEI results for tablets stored in low oxygen content foil pouches (blister packs) were consistently lower than for tablets stored in packages containing normal atmospheric conditions or at higher oxygen environments. This was true for each of the stability conditions (25 °C, 60%RH, 30 °C, 65%RH and 40 °C, 75%RH).

To examine the impact of moisture content on resultant tablet stability in nitrogen inerted or normal atmosphere blisters, tablets of formulation 2 (*infra*) were stored at different relative humidities and then packaged into cold form blisters, with or without nitrogen inerting. The results are presented in the Table 3.

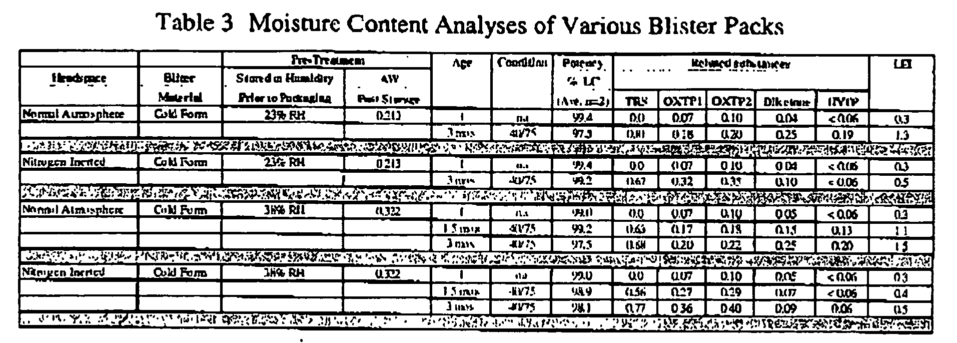

As shown in Table 3, the potency of tablets exposed to lower humidities prior to packaging and then nitrogen inerted when placed into cold form aluminum blisters was consistently higher than for tablets exposed to higher humidities and then packaged under normal atmospheric conditions. This was true for each of the stability conditions (25 °C, 60%RH, 30 °C, 65%RH and 40 °C, 75%RH).

Also as shown in Table 3, the LEI's in tablets exposed to lower humidities prior to packaging and then nitrogen inerted when placed into cold form aluminum blisters were consistently lower than for tablets exposed to higher humidities and then packaged under normal atmospheric conditions. This was true for each of the stability conditions (25 °C, 60%RH, 30 °C, 65%RH and 40 °C, 75%RH).

### Diketone Peak

With respect to the assayed level of the diketone related substance peak, the invention also demonstrates a significant, unexpected and advantageous improvement over other forms of formulation. Each study (tables 1-3) illustrated that the formation of this impurity was related to oxygen concentrations. Packages such as the cold form nitrogen inerted aluminum foil blister packs containing and maintaining lower oxygen environments tended to minimize the formation of this impurity.

### HYTP Peak

With respect to the amount of the HYTP peak, the invention demonstrates a significant, unexpected and advantageous improvement over other forms of formulation/packaging. Each study (Tables 1-3) illustrated that the formation of this impurity was related to oxygen concentrations. Packages such as cold form nitrogen inerted aluminum foil blister packs containing and maintaining lower oxygen content tended to minimize the formation of this impurity. In addition, as this impurity is derived from hydrolysis products (OXTP)- exposure to lower relative humidities and/or packages containing a desiccant also helped to lessen the formation of this impurity.

Thus, applicants have also provided a method of advantageously controlling the distribution of impurities. The use of nitrogen-inerted blister packs reduces the amounts of impurities as discussed above. Importantly, it also has the effect of shifting the distribution of impurities by particularly minimizing the amount of LEI's. Applicants by operation of the present invention have achieved control of water activity to levels from about 0.2 to about 0.4. Also applicants have achieved reductions in oxygen content in the head space of the blister pack to about less than 2% to 4%.

### Method of Using the Invention

The method of using the invention involves preparing and administering a pharmaceutical formulation comprising tablets, caplets or capsules of the compound of formula I packaged in nitrogen-inerted aluminum blister packs. The blister packs may be individual units or a pallet of multiple blister packs joined at appropriate perforation points for ease of dispensing or packaging for sale as appropriate or approved. The method of the invention involves formulation of the active ingredient into a tablet, caplet or capsule including slow release capsule or fast disintegrating tables packaged in gas-inerted blister packs preferably aluminum foil blister packs. The improved formulation as defined herein includes the packaging of the tablet, caplet or capsule into a nitrogen inerted blister pack, preferably aluminum foil blister packs. Typically, the tablet, caplet or capsule may contain from about 1 to about 60 mg of the compound of formula I. Preferably the tablet, caplet or capsule may contain from about 5 mg to about 60 mg base equivalents of the compound of formula I. Most preferably, the tablet, caplet or capsule contains about 5 mg, 10 mg, 15 mg, 30 mg, or 60 mg base equivalents of the compound of formula I.

The following procedures of making the tablet, caplet or capsule useful for the practice of the invention are illustrative only and are not intended to limit the scope of the invention in any way. It is understood that the tablets, caplets or capsules so made are then packaged in nitrogen-inerted aluminum foil blister packs the preparation of which is described in the examples and elsewhere in this document. "Active ingredient", refers to a compound according to formula (I) or a pharmaceutically acceptable salt, solvate, active metabolite, enantiomer, racemate or prodrug thereof with or without other cardio protective agent(s) which is/are to be administered to a patient in need thereof, optionally in combination with aspirin or as an adjunct to a stent or PCI procedure.

### Examples

The following per tablet, caplet or capsule formulation examples, and reference examples are intended to further illustrate the present invention and are not intended to limit the scope of this invention.

### Formulation 1

CS-747 HCl (13.72 mg equivalent to 12.5 mg base), mannitol, hydroxypropyl methylcellulose, croscarmellose sodium, microcrystalline cellulose and magnesium stearate are blended and then roller compacted to produce a granulation. To the resulting granulation, additional croscarmellose sodium, microcrystalline cellulose and magnesium stearate are added and the material is blended and compressed to form tablets weighing 250 mg. An Opadry^{®} II beige film coating mixture is added to water and then sprayed onto these tablets in a side vented coating pan.
The tablet is then packaged in an aluminum foil blister pack, inerted or filled with a gas such as nitrogen and then sealed using procedures known to one of skill in the art.

### Formulation 2

CS-747 HCl (10.98 mg equivalent to 10.00 mg base) mannitol, hydroxypropyl methylcellulose, croscarmellose sodium, microcrystalline cellulose and magnesium stearate are blended and then roller compacted to produce a granulation. To the resulting granulation, additional croscarmellose sodium, microcrystalline cellulose and magnesium stearate are added and the material is blended and compressed to form tablets weighing 250 mg. An Opadry II^{®} beige film coating mixture is added to water and then sprayed onto these tablets in a side vented coating pan.

Solid compositions of formula I may be prepared using the ingredients below per tablet, capsule or caplet:

### Formulation 3

CS-747 HCl (5.49 mg equivalent to 5.0 mg base), mannitol, hydroxypropyl methylcellulose, croscarmellose sodium, microcrystalline cellulose and magnesium stearate are blended and then roller compacted to produce a granulation(s). To the resulting granulation(s), additional croscarmellose sodium, microcrystalline cellulose and magnesium stearate are added and the material is blended and compressed to form tablets weighing from 125-250 mg. An Opadry^{®} II beige film coating mixture is added to water and then sprayed onto these tablets in a side vented coating pan.

The resulting tablet(s), caplet(s), or capsule(s) are then packaged in a nitrogen-inerted blister pack(s) using procedures disclosed herein and/or known to one of skill in the art or attained with minimal experimentation by one of skill in the art. The tablet(s), caplet(s), or capsule(s) are then placed in boxes for storage and/or shipping.

### Formulation 4

CS-747 HCl (8.24 mg equivalent to 7.5 mg base), mannitol, hydroxypropyl methylcellulose, croscarmellose sodium, microcrystalline cellulose and magnesium stearate are blended and then roller compacted to produce a granulation(s). To the resulting granulation(s), additional croscarmellose sodium, microcrystalline cellulose and magnesium stearate are added and the material is blended and compressed to form tablets weighing from 125-250 mg. An Opadry II^{®} beige film coating mixture is added to water and then sprayed onto these tablets in a side vented coating pan. The resulting tablet(s), caplet(s), or capsule(s) are then packaged in a nitrogen-inerted blister pack(s) using procedures disclosed herein and/or known to one of skill in the art or attained with minimal experimentation by one of skill in the art. The tablct(s), caplet(s), or capsule(s) are then placed in boxes for storage and/or shipping.

### Formulation 5

CS-747 HCl (16.47 mg equivalent to 15.00 mg base), mannitol, hydroxypropyl methylcellulose, croscarmellose sodium, microcrystalline cellulose and magnesium stearate are blended and then roller compacted to produce a granulation(s). To the resulting granulation(s), additional croscarmellose sodium, microcrystalline cellulose and magnesium stearate are added and the material is blended and compressed to form tablets weighing from 125-250 mg. An Opadry II^{®} beige film coating mixture is added to water and then sprayed onto these tablets in a side vented coating pan.
The resulting tablet(s), caplet(s), or capsule(s) are then packaged in a nitrogen-inerted blister pack(s) using procedures disclosed herein and/or known to one of skill in the art or attained with minimal experimentation by one of skill in the art. The tablet(s), caplet(s), or capsule(s) are then placed in boxes for storage and/or shipping.

### Formulation 6

CS-747 HCl (32.94 mg equivalent to 30 mg base), mannitol, hydroxypropyl methylcellulose, croscarmellose sodium, microcrystalline cellulose and magnesium stearate are blended and then roller compacted to produce a granulation(s). To the resulting granulation(s), additional croscarmellose sodium, microcrystalline cellulose and magnesium stearate are added and the material is blended and compressed to form tablets weighing from 125-250 mg. An Opadry II^{®} beige film coating mixture is added to water and then sprayed onto these tablets in a side vented coating pan.
The resulting tablet(s), caplet(s), or capsule(s) are then packaged in a nitrogen-inerted blister pack(s) using procedures disclosed herein and/or known to one of skill in the art or attained with minimal experimentation by one of skill in the art. The tablet(s), caplet(s), or capsule(s) are then placed in boxes for storage and/or shipping.

One of skill in the art is aware that other doses of the compound of formula I such as, for example 60 mg dose may be prepared following the procedures outlined above for given doses with appropriate adjustments as necessary.

## Claims

1. A formulation comprising a therapeutically effective amount of the compound of formula I 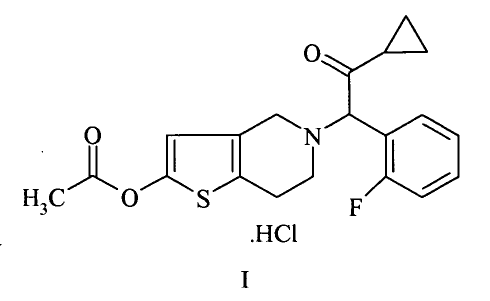 packaged in an air and moisture impervious gas-inerted blister pack.

2. A formulation according to Claim 1 wherein the therapeutically effective amount of the compound of formula I is about 5 mg to about 60 mg base equivalent.

3. A formulation according to Claim 1 wherein the therapeutically effective amount of the compound of formula I is about 5 mg base equivalent.

4. A formulation according to Claim 1 wherein the therapeutically effective amount of the compound of formula I is about 10 mg base equivalent.

5. A formulation according to Claim 1 wherein the therapeutically effective amount of the compound of formula I is about 15 mg base equivalent.

6. A formulation according to Claim 1 wherein the therapeutically effective amount of the compound of formula I is about 60 mg base equivalent.

7. A formulation according Claim 1 wherein the blister pack is an aluminum foil blister pack.

8. A formulation according to Claim 1 wherein the formulation comprises a tablet, caplet or capsule comprising a compound of formula I.

9. A formulation according to Claim 1 wherein the inerting gas is nitrogen.

10. A process for the manufacture of a compound of formula I comprising the steps of:
a. preparing tablets, caplets or capsules of the compound of formula I, and
b. packaging said tablets, caplets, capsules or other solid formulation of the compound of formula I in gas inerted blister packs.

11. A process according to Claim 10 wherein oxygen content in the head space of the blister pack is reduced to about less than 2% to 4.0 %, and the water activity in the tablets, caplets or capsules of the compound of formula I is controlled to less than about 0.2 to 0.4.

12. An article of manufacture comprising a compound of formula I packaged in an air and moisture impervious gas-inerted blister pack.

13. A formulation of the compound of formula I comprising a tablet, caplet, capsule or other solid formulation of the compound of formula I which has been packaged in an air and moisture impervious gas-inerted blister pack for use as a medicament.

14. A formulation of the compound of formula I comprising a tablet, caplet, capsule or other solid formulation of the compound of formula I which has been packaged in an air and moisture impervious gas-inerted blister pack for use in the treatment of thrombosis, acute coronary syndrome, acute coronary syndrome - medically managed, stroke, cerebrovascular aneurysyms and high risk vascular diseases.

## Patentansprüche

1. Formulierung, umfassend eine therapeutisch wirksame Menge der Verbindung der Formel I 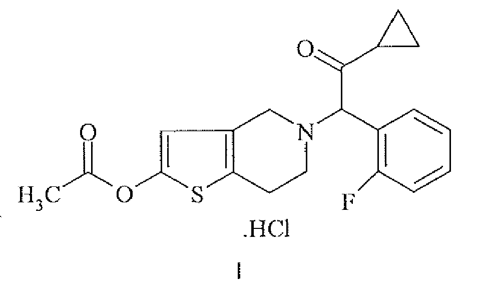 die in einer luft- und feuchtigkeitsundurchlässigen Gas-inertierten Blisterpackung abgepackt ist.

2. Formulierung nach Anspruch 1, worin die therapeutisch wirksame Menge der Verbindung der Formel I etwa 5 mg bis etwa 60 mg Basenäquivalente beträgt.

3. Formulierung nach Anspruch 1, worin die therapeutisch wirksame Menge der Verbindung der Formel I etwa 5 mg Basenäquivalente beträgt.

4. Formulierung nach Anspruch 1, worin die therapeutisch wirksame Menge der Verbindung der Formel I etwa 10 mg Basenäquivalente beträgt.

5. Formulierung nach Anspruch 1, worin die therapeutisch wirksame Menge der Verbindung der Formel I etwa 15 mg Basenäquivalente beträgt.

6. Formulierung nach Anspruch 1, worin die therapeutisch wirksame Menge der Verbindung der Formel I etwa 60 mg Basenäquivalente beträgt.

7. Formulierung nach Anspruch 1, worin die Blisterpackung eine Aluminiumfolie-Blisterpackung ist.

8. Formulierung nach Anspruch 1, worin die Formulierung eine Tablette, Caplette oder Kapsel umfasst, die eine Verbindung der Formel umfasst.

9. Formulierung nach Anspruch 1, worin das inertierende Gas Stickstoff ist.

10. Verfahren zur Herstellung einer Verbindung der Formel I, umfassend die folgenden Stufen:
a. Herstellung von Tabletten, Capletten oder Kapseln der Verbindung der Formel I, und
b. Abpackung der Tabletten, Capletten, Kapseln oder einer sonstigen festen Formulierung der Verbindung der Formel I in Gas-inertierte Blisterpackungen

11. Verfahren nach Anspruch 10, worin der Sauerstoffgehalt im Kopfraum der Blisterpackung auf weniger als etwa 2% bis 4,0 % reduziert wird und die Wasseraktivität der Tabletten, Capletten oder Kapseln der Verbindung der Formel I auf weniger als etwa 0,2 bis 0,4 gesteuert wird.

12. Herstellungsgegenstand, umfassend eine Verbindung der Formel I, die in eine luft- und feuchtigkeitsundurchlässige Gas-inertierte Blisterpackung abgepackt ist.

13. Formulierung der Verbindung der Formel I, umfassend eine Tablette, Caplette, Kapsel oder sonstige feste Formulierung der Verbindung der Formel I, die in eine luft- und feuchtigkeitsundurchlässige Gas-inertierte Blisterpackung abgepackt ist, zur Verwendung als ein Arzneimittel.

14. Formulierung der Verbindung der Formel I, umfassend eine Tablette, Caplette, Kapsel oder sonstige feste Formulierung der Verbindung der Formel I, die in eine luft- und feuchtigkeitsundurchlässige Gas-inertierte Blisterpackung abgepackt ist, zur Verwendung bei der Behandlung von Thrombose, akutem Koronarsyndrom, akutem Koronarsyndrom, das medikamentös behandelt wird, Hirnschlag, zerebrovaskulären Aneurysmen und Vaskulärkrankheiten mit hohem Risiko.

## Revendications

1. Formulation comprenant une quantité thérapeutiquement efficace du composé répondant à la formule I 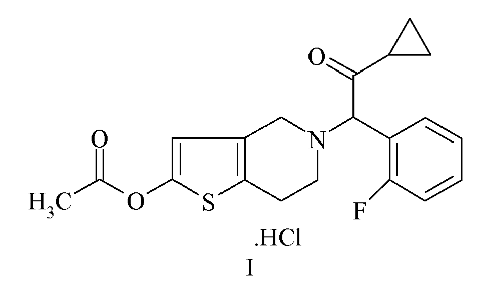 conditionnée dans une plaquette contenant un gaz inerte, imperméable à l'air et à l'humidité.

2. Formulation selon la revendication 1, dans laquelle la quantité thérapeutiquement efficace du composé répondant à la formule I s'élève d'environ 5 mg à environ 60 mg d'équivalent de base.

3. Formulation selon la revendication 1, dans laquelle la quantité thérapeutiquement efficace du composé répondant à la formule I s'élève à environ 5 mg d'équivalent de base.

4. Formulation selon la revendication 1, dans laquelle la quantité thérapeutiquement efficace du composé répondant à la formule I s'élève à environ 10 mg d'équivalent de base.

5. Formulation selon la revendication 1, dans laquelle la quantité thérapeutiquement efficace du composé répondant à la formule I s'élève à environ 15 mg d'équivalent de base.

6. Formulation selon la revendication 1, dans laquelle la quantité thérapeutiquement efficace du composé répondant à la formule I s'élève à environ 60 mg d'équivalent de base.

7. Formulation selon la revendication 1, dans laquelle la plaquette est une plaquette en feuille d'aluminium.

8. Formulation selon la revendication 1, dans laquelle la formulation comprenant un comprimé, un comprimé en forme de capsule ou une capsule comprenant un composé répondant à la formule I.

9. Formulation selon la revendication 1, dans laquelle le gaz qui rend inerte est de l'azote.

10. Procédé pour la préparation d'un composé répondant à la formule I, comprenant les étapes :
a. de préparation de comprimés, de comprimés en forme de capsules ou de capsules du composé répondant à la formule I ; et
b. de conditionnement desdits comprimés, desdits comprimés en forme de capsules, desdites capsules ou d'une autre formulation solide du composé répondant à la formule I dans des plaquettes contenant un gaz inerte.

11. Procédé selon la revendication 10, dans lequel la teneur en oxygène dans l'espace de tête de la plaquette est réduite à concurrence d'une quantité inférieure à une valeur d'environ 2 % à 4,0 %, et l'activité de l'eau dans les comprimés, les comprimés en forme de capsules ou les capsules du composé répondant à la formule I est réglée pour être inférieure à une valeur d'environ 0,2 à 0,4.

12. Article de fabrication comprenant un composé répondant à la formule I conditionné dans une plaquette contenant un gaz inerte, imperméable à l'air et à l'humidité.

13. Formulation du composé répondant à la formule I, comprenant un comprimé, un comprimé en forme de capsule, une capsule ou une autre formulation solide du composé répondant à la formule I qui a été conditionnée dans une plaquette contenant un gaz inerte, imperméable à l'air et à l'humidité à utiliser comme médicament.

14. Formulation du composé répondant à la formule I, comprenant un comprimé, un comprimé en forme de capsule, une capsule ou une autre formulation solide du composé répondant à la formule I qui a été conditionnée dans une plaquette contenant un gaz inerte, imperméable à l'air et à l'humidité à utiliser dans le traitement de la thrombose, du syndrome coronarien aigu, du syndrome coronarien aigu géré par voie médicale, d'un accident vasculaire cérébral, d'anévrismes cérébrovasculaires et de maladies vasculaires à haut risque.
